# EUROPÄISCHE PATENTSCHRIFT

(11) **EP 1 314 446 B1**
(45) Veröffentlichungstag und Bekanntmachung des Hinweises auf die Patenterteilung: **07.06.2006**
(21) Anmeldenummer: 02018878.5
(22) Anmeldetag: 24.08.2002
(51) Int. Cl.: A61M 16/06, A61M 16/08

(54) **Beatmungsmaske**
Breathing mask
Masque respiratoire

(30) Priorität: 27.11.2001 DE 10158066
(43) Veröffentlichungstag der Anmeldung: 28.05.2003
(73) Patentinhaber: Weinmann Geräte für Medizin GmbH & Co. KG, 22525 Hamburg (DE)
(72) Erfinder: Eifler, Martin, 25348 Glückstadt (DE); Schulz, Gerd, 22869 Schenefeld (DE)
(74) Vertreter: Klickow, Hans-Henning

(56) Entgegenhaltungen:
- DE-U- 29 723 101
- US-A- 5 937 851

## Beschreibung

Die Erfindung betrifft eine Beatmungsmaske für die Beatmung eines Patienten, bei der ein Maskengrundkörper über ein Kupplungselement mit einem Ausatmungselement verbunden ist, das einen spaltartigen Ausströmkanal begrenzt und bei der das Kupplungselement einen Halterungsstutzen zur Positionierung des Ausatmungselementes aufweist, sowie ein das Kupplungselement mit dem Maskengrundkörper verbindendes Gelenk von einem Übergangssegment mit dem Halterungsstutzen verbunden ist und bei der das Übergangssegment mindestens bereichsweise geschwungen verläuft, sowie bei der das Ausatmungselement mindestens bereichsweise gemeinsam mit dem Kupplungselement den Ausströmkanal begrenzt, das Kupplungselementüber ein Gelenk mit dem Maskengrundkörper verbunden ist, das Ausatmungselement innerhalb des Kupplungselementes von mindestens einem Arretierungselement fixiert ist, das mit einer Rastnase einen zugeordneten Absatz hintergreift und bei der der Absatz im Bereich einer den Halterungsstutzen mit dem Übergangssegment verbindenden Überleitung angeordnet ist, dadurch gekennzeichnet, daß das Gelenk kugelsegmentartig ausgebildet ist, daß das Ausatmungselement von mehreren Arretierungselementen fixiert ist und daß die Arretierungsflächen der Rastnasen relativ zu einer Längsachse des Ausatmungselementes derart geneigt angeordnet sind, daß eine Steigung ausgehend von einer Innenfläche des Halterungsstutzen in Richtung auf das Übergangssegment bereitgestellt ist.

Eine Beatmungsmaske mit Ausatmungselement wird beispielsweise in der DE-OS 199 03 732 beschrieben. Der Maskengrundkörper ist hierbei starr in ein Kupplungselement übergeleitet, das mit dem Ausatmungselement koppelbar ist. Das Ausatmungselement ist mit einem Schlauchadapter verbindbar, der die Atemmaske über einen Beatmungsschlauch an ein Beatmungsgerät anschließt. Eine derartige Beatmungsmaske ist insbesondere dafür geeignet, bei der sogenannten CPAP-Therapie eingesetzt zu werden. Ein Ausströmelement mit spaltartigem Ausströmkanal ist aus der US-PS 59 37 851 bekannt.

Derartige Beatmungsmasken bestehen einschließlich des Kupplungselementes, des Ausatmungselementes sowie der Anschlußteile für den Beatmungsschlauch aus einer Vielzahl von Bauelementen, die zu relativ hohen Fertigungspreisen beitragen.

Bekannt ist es ebenfalls, ein Ausatemsystem im Bereich einer Atemmaske derart zu realisieren, daß direkt am Maskengrundkörper mehrere Ausatemöffnungen angeordnet sind. Hierdurch werden jedoch relativ laute Ausatemgeräusche unmittelbar im Kopfbereich des Patienten erzeugt. Dies ist insbesondere bei Anwendungen während der Nacht nicht akzeptabel.

Aus der US-A-5,937,851 ist bereits eine Beatmungsmaske gemäß Oberbegriff von Anspruch 1 für die Beatmung eines Patienten bekannt, bei der ein Maskengrundkörper über ein Kupplungselement mit einem Ausatmungselement verbunden ist. Das Ausatmungselement begrenzt gemeinsam mit dem Kopplungselement einen spaltartigen Ausströmkanal und das Kupplungselement weist einen Halterungsstutzen zur Positionierung des Ausatmungselementes auf. Der Halterungsstutzen ist von einem rechtwinkligen Rohrstück mit dem Maskengrundkörper verbunden.

Aus der DE 297 23 101 U ist es bekannt, ein kugelsegmentartiges Gelenk zur Verbindung eines Maskengrundkörpers mit einem Beatmungsschlauch zu verwenden. Das Kugelgelenk ist über ein rechtwinkliges Rohrstück und ein zylinderartiges Anschlußstück mit dem Beatmungsschlauch verbunden.

Aufgabe der vorliegenden Erfindung ist es, eine Beatmungsmaske der einleitend genannten Art derart zu konstruieren, daß eine geringe Schallemission beim Ausatmen bei geringer Teileanzahl und hohem Benutzungskomfort erreicht wird.

Diese Aufgabe wird erfindungsgemäß dadurch gelöst, daß das Gelenk kugelsegmentartig ausgebildet ist, daß das Ausatmungselement von mehreren Arretierungselementen fixiert ist und daß die Arretierungsflächen der Rastnasen relativ zu einer Längsachse des Ausatmungselementes derart geneigt angeordnet sind, daß eine Steigung ausgehend von einer Innenfläche des Halterungsstutzen in Richtung auf das Übergangssegment bereitgestellt ist.

Durch die Verbindung des Kupplungselementes über das kugelsegmentartige Gelenk mit dem Maskengrundkörper wird eine optimale Bewegungsfreiheit des Schlauchsystems an der Maske bereitgestellt. Durch die Ausströmung der Atemluft durch den spaltartigen Ausströmkanal hindurch wird ein relativ leises Ausströmen der Luft ermöglicht und durch die Begrenzung des Ausströmkanals sowohl durch das Kupplungselement als auch durch das Ausatmungselement wird eine geringe Teileanzahl unterstützt.

Eine geringe Anzahl von Bauelementen wird dadurch erreicht, daß das Kupplungselement einen Halterungsstutzen zur Positionierung des Ausatmungselementes aufweist.

Zur Unterstützung einer für den Patienten angenehmen Anordnung der benötigen Funktionskomponenten wird vorgeschlagen, daß der Halterungsstutzen relativ zum Maskengrundkörper angewinkelt angeordnet ist.

Ein vorteilhafter Abstand des Ausatmungselementes zu einer Gesichtsfläche des Patienten wird dadurch vorgegeben, daß das Gelenk von einem Übergangssegment mit dem Halterungsstutzen verbunden ist.

Zur Unterstützung einer einfachen Entformbarkeit bei einer spritzgußtechnischen Herstellung der Bauteile ist vorgesehen, daß das Übergangssegment mindestens bereichsweise gebogen verläuft.

Eine funktionssichere und einfach zu handhabende Halterung kann dadurch bereitgestellt werden, daß das Ausatmungselement mindestens bereichsweise innerhalb des Halterungsstutzens anordbar ist.

Eine zuverlässige Fixiermöglichkeit für das Ausatmungselement wird dadurch bereitgestellt, daß das Ausatmungselement innerhalb des Kupplungselementes von mindestens einem Arretierungselement fixiert ist, das mit einer Rastnase einen zugeordneten Absatz hintergreift.

Eine einfache Geometrie der verwendeten Bauelemente wird dadurch unterstützt, daß der Absatz im Bereich einer den Halterungsstutzen mit dem Übergangssegment verbindenden Überleitung angeordnet ist.

Zur Vermeidung einer Beschädigung der Arretierungselemente ist vorgesehen, daß das Ausatmungselement außenseitig mindestens einen Außensteg aufweist, der eine maximale Positioniertiefe des Ausatmungselementes innerhalb des Kupplungselementes definiert.

Ein vorteilhaftes Ausströmverhalten wird dadurch unterstützt, daß der spaltartige Ausströmkanal zwischen einem Grundsegment des Ausatmungselementes und dem Halterungsstutzen angeordnet ist.

Ein Anschluß der Beatmungsmaske an ein Beatmungsgerät unter Verwendung möglichst weniger Bauelemente wird dadurch erreicht, daß das Ausatmungselement mit einem Verbindungsschlauch koppelbar ist.

Ein günstiges Ausströmverhalten bei einer zugleich kompakten geometrischen Gestaltung des Ausströmelementes wird dadurch unterstützt, daß das Grundsegment des Ausatmungselementes von mindestens zwei Verbindungsstegen mit einem Halterungssegment verbunden ist und daß sich zwischen den Verbindungsstegen mindestens eine Ausströmöffnung erstreckt.

Eine Trennung der Bauelemente voneinander wird dadurch ermöglicht, daß die Rastnase eine geneigt zur Längsachse verlaufende Arretierungsfläche aufweist und daß der Absatz gleichfalls geneigt zur Längsachse verläuft.

Zur Erleichterung einer Beweglichkeit des Ausatmungselementes relativ zum Halterungsstutzen wird vorgeschlagen, daß sich der Absatz im wesentlichen konzentrisch relativ zur Längsachse erstreckt.

Eine Einleitung von Kräften in die Beatmungsmaske, die vom Verbindungsschlauch übertragen werden, kann dadurch vermieden oder zumindest reduziert werden, daß das Ausatmungselement drehbeweglich innerhalb des Halterungsstutzens geführt ist.

Zur Bereitstellung eines relativ zur Längsachse radial umlaufenden im wesentlichen gleichmäßig breiten Ausatmungsspaltes wird vorgeschlagen, daß das Ausatmungselement relativ zum Halterungsstutzen zentriert ist.

Ein ungewolltes Verschließen des Ausströmspaltes durch einen auf das Ausatmungselement aufgeschobenen Verbindungsschlauch kann dadurch vermieden werden, daß die Außenstege einen Anschlag für einen auf das Ausatmungselement aufgeschobenen Verbindungsschlauch bereitstellen.

In den Zeichnungen sind Ausführungsbeispiele der Erfindung schematisch dargestellt. Es zeigen:
- Fig. 1: Eine schematische Darstellung einer Beatmungseinrichtung mit Beatmungsmaske,
- Fig. 2: eine vergrößerte Darstellung der Beatmungsmaske mit kugelgelenkartig angekoppeltem Kupplungselement,
- Fig. 3: das Kupplungselement gemäß Fig. 2 mit herausgezogenem Ausatmungselement und
- Fig. 4: einen Längsschnitt durch einen Halterungsstutzen des Kupplungselementes, in den das Ausatmungselement zur Begrenzung des Ausströmkanals eingeführt ist.

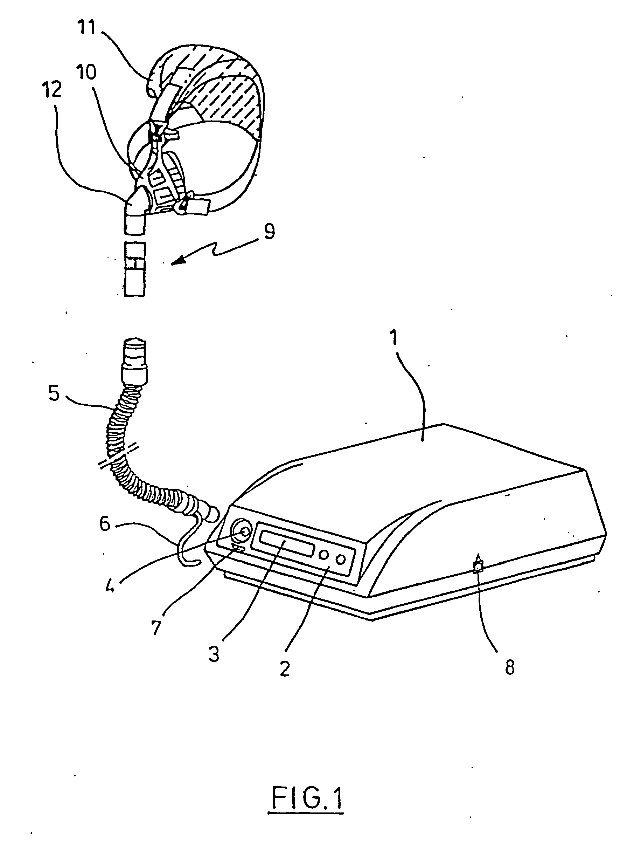

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

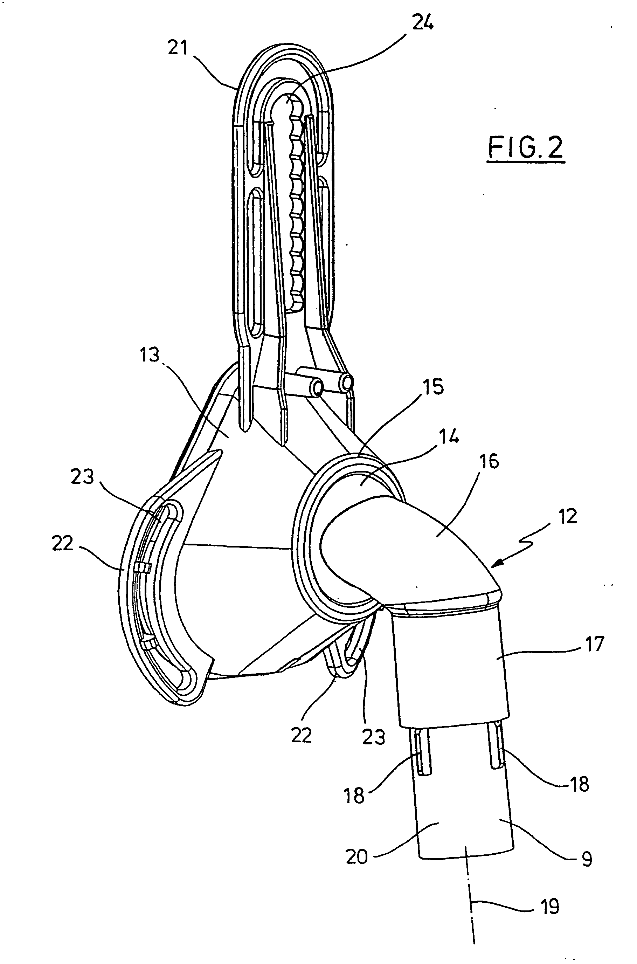

Fig. 2 zeigt die Beatmungsmaske (10) in einer vergrößerten perspektivischen Darstellung. Es ist zu erkennen, daß ein Maskengrundkörper (13) über ein kugelsegmentartiges Gelenk (14) mit dem Kupplungselement (12) verbunden ist. Das Gelenk (14) ist dabei in eine Ausnehmung (15) des Maskengrundkörpers (13) eingeführt. Ausgehend vom Gelenk (14) erstreckt sich das Kupplungselement (12) zunächst mit einem gebogenen kubusartigen Übergangssegment (16), das im Bereich seiner dem Gelenk (14) abgewandten Ausdehnung mit einem Halterungsstutzen (17) verbunden ist.

Bei der in Fig. 2 dargestellten Ausführungsform ist das Ausatmungselement bereichsweise in den Halterungsstutzen (17) eingeführt. Eine maximale Einführtiefe wird durch Außenstege (18) vorgegeben, die im wesentlichen parallel zu einer Längsachse (19) des Ausatmungselementes (9) entlang einer Außenseite (20) des Ausatmungselementes (9) verlaufen.

Der Maskengrundkörper (13) ist darüber hinaus mit einem Halterungssteg (21) sowie mit Seitenflanschen (22) verbunden. Im Bereich der Seitenflansche (22) sind Ausnehmungen (23) angeordnet, die zur Befestigung der in Fig. 1 dargestellten Kopfhaube (11) dienen. Der Halterungssteg (21) weist eine Ausnehmung (24) auf, innerhalb derer eine nicht dargestellte Halterungseinrichtung für eine Stirnstütze positioniert werden kann. Eine definierte Positionierung der Stirnstütze kann hierbei durch eine Rastung innerhalb der Ausnehmung (24) unterstützt werden.

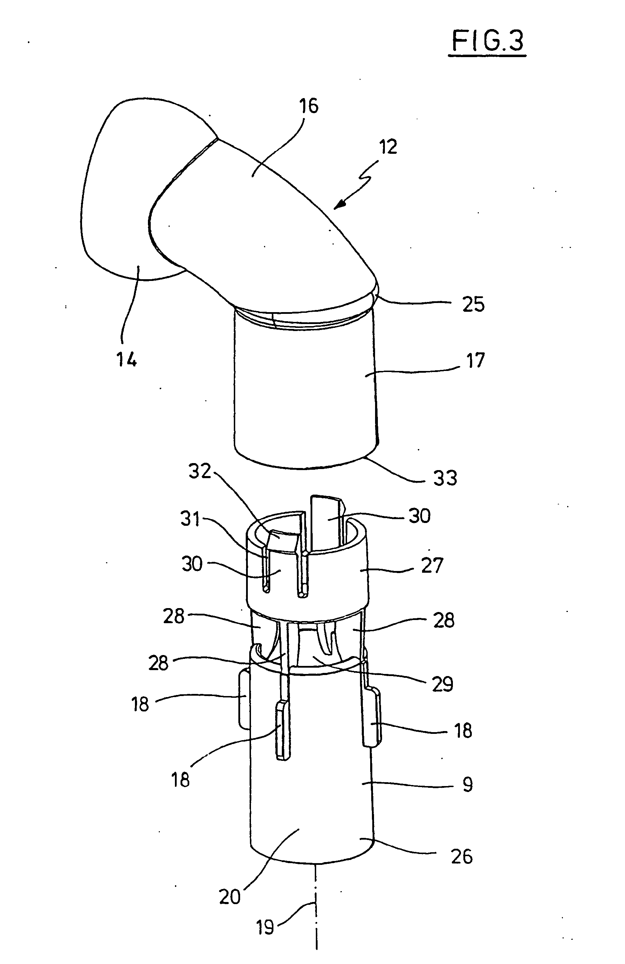

Fig. 3 zeigt das Kupplungselement (12) gemäß Fig. 2 mit aus dem Halterungsstutzen (17) herausgezogenem Ausatmungselement (9). Es ist insbesondere die kugelsegmentartige Ausbildung des Gelenks (14) sowie der geschwungene Verlauf des Übergangsegmentes (16) zu erkennen. Darüber hinaus ist zu erkennen, daß der Halterungsstutzen (17) einen geringeren Außendurchmesser aufweist, als das Übergangssegment (16) im Bereich einer Überleitung (25) in den Halterungsstutzen (17).

Das Ausatmungselement (9) besteht im wesentlichen aus einem Grundsegment (26) und einem Halterungselement (27), die durch Verbindungsstege (28) miteinander gekoppelt sind. Zwischen den Verbindungsstegen (28) erstrecken sich Ausströmöffnungen (29). Bei der dargestellten' Ausführungsform sind sowohl das Grundsegment (26) als auch das Halterungssegment (27) im wesentlichen ringförmig ausgebildet und konzentrisch zur Längsachse (19) angeordnet.

Das Halterungssegment (27) ist mit Arretierungselementen (30) versehen, die ähnlich zu federnden Zungen konstruiert und mit Rastnasen (31) sowie Einführanschrägungen (32) versehen sind. Bei einem Einschieben des Ausatmungselementes (9) in den Halterungsstutzen (17) werden hierdurch beim Kontakt des Halterungsstutzens (17) mit den Einführanschrägungen (32) die Arretierungselemente (30) federnd zurückgeschoben und das Ausatmungselement (9) kann innerhalb des Halterungsstutzens (17) positioniert werden. Das Ausatmungselement (9) kann hierbei soweit in den Halterungsstutzen (17) eingeführt werden, bis die Außenstege (18) gegen einen Rand (33) des Halterungsstutzens (17) stoßen. In diesem Zustand federn die Arretierungselemente (30) mit ihren Rastnasen (31) in den Bereich der Überleitung (25) und führen hier eine Fixierung des Ausatmungselementes (9) durch. Die Außenstege (18) dienen ebenfalls als Anschlag für den Verbindungsschlauch (5), wenn dieser auf das Ausströmelement (9) aufgeschoben wird. Hierdurch kann ein ungewolltes Verschließen des Ausströmspaltes durch den aufgeschobenen Verbindungsschlauch vermieden werden.

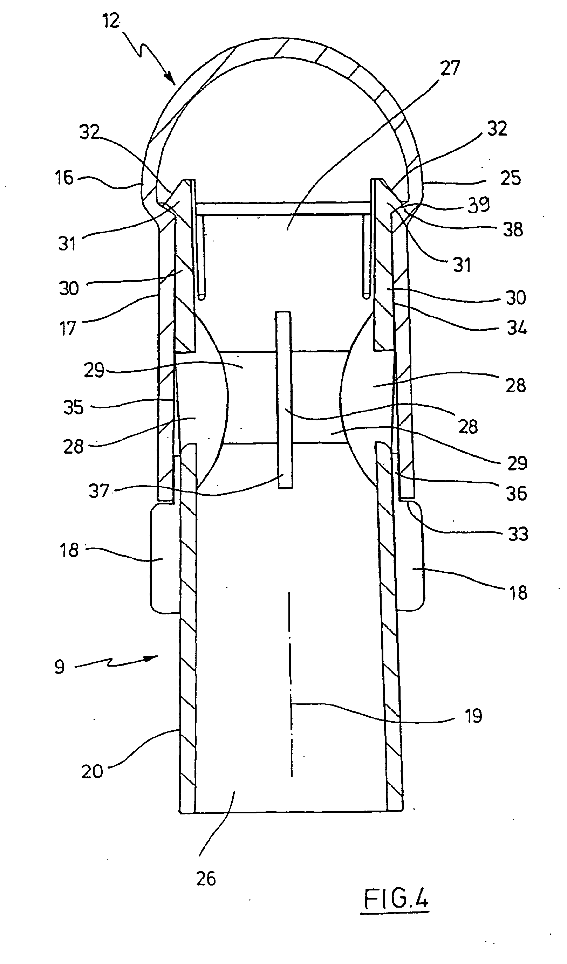

Fig. 4 zeigt eine teilweise Darstellung eines Längsschnittes durch ein Kupplungselement (12), in das ein Ausatmungselement (9) eingeführt ist. Zu erkennen ist insbesondere, daß das Halterungssegment (27) mit einer Außenfläche (34) im wesentlichen an einer Innenfläche (35) des Halterungsstutzens (17) anliegt. Ein Außendurchmesser des Halterungssegmentes (27) wird dabei nur so viel kleiner als ein Innendurchmesser des Halterungsstutzens (17) gewählt, daß auch unter Berücksichtung von Fertigungstoleranzen ein leichtgängiges Hineinschieben des Ausatmungselementes (9) in den Halterungsstutzen (17) hinein möglich ist.

Ein Außendurchmesser des Grundsegmentes (26) ist relativ zu einem Innendurchmesser des Halterungsstutzens (17) so viel kleiner ausgebildet, daß ein Ausströmspalt (36) bereitgestellt ist.

Als Verlängerung der Außenstege (18) in Richtung auf die Verbindungsstege (28) können im Material des Ausatmungselementes (9) Außenrippen (37) angeordnet sein, die beispielsweise als flache Stege realisiert sein können und die zu einer Zentrierung des Ausatmungselementes (9) und des Halterungsstutzens (17) relativ zur Längsachse (19) dienen, um einen näherungsweise gleichmäßig breit umlaufenden Ausströmspalt zu gewährleisten. Die Rastnasen (31) hintergreifen einen Absatz (38), der innenseitig im Bereich der Überleitung (25) angeordnet ist und führen hierdurch eine Arretierung des Ausatmungselementes (9) relativ zum Kupplungselement (12) durch.

Die Querschnittdarstellung in Fig. 4 veranschaulicht, daß die Absätze (38) relativ zur Längsachse (19) geneigt angeordnet sind. Hierdurch wird eine Steigung ausgehend von der Innenfläche (35) des Halterungsstutzens (17) in Richtung auf das Übergangssegment (16) bereitgestellt. Die Rastnase (31) ist mit einer Arretierungsfläche (39) versehen, die im wesentlichen eine gleiche räumliche Orientierung wie der Absatz (38) aufweist. Bei Erzeugung einer vorgegebenen Mindestzugkraft wird hierdurch das Ausatmungselement (9) aus dem Halterungsstutzen (17) herausgezogen, da die Rastnasen (31) mit ihren Arretierungsflächen (39) am Absatz (38) entlang gleiten und hierdurch ein Zurückfedern der Arretierungselemente (30) in Richtung auf die Längsachse (19) vorgegeben wird.

Durch das Spiel zwischen dem Halterungsstutzen (17) und dem Halterungssegment (27) ist es auch möglich, eine einfache Drehbarkeit des Ausatmungselementes (9) innerhalb des Halterungsstutzens (17) zu unterstützen. Zu dieser einfachen Drehbarkeit trägt ebenfalls bei, daß sich der Absatz (38) im wesentlichen konzentrisch zur Längsachse (19) erstreckt. Durch die drehbare Lagerung des Ausatmungselementes (9) innerhalb des Halterungsstutzens (17) wird eine vom Verbindungsschlauch (5) verursachte Krafteinleitung in die Beatmungsmaske (10) vermieden.

## Patentansprüche

1. Beatmungsmaske für die Beatmung eines Patienten, bei der ein Maskengrundkörper (13) über ein Kupplungselement (12) mit einem Ausatmungselement (9) verbunden ist, das einen spaltartigen Ausströmkanal begrenzt und bei der das Kupplungselement (12) einen Halterungsstutzen (17) zur Positionierung des Ausatmungselementes (9) aufweist, sowie ein das Kupplungselement (12) mit dem Maskengrundkörper (13) verbindendes Gelenk (14) von einem Übergangssegment (16) mit dem Halterungsstutzen (17) verbunden ist und bei der das Übergangssegment (16) mindestens bereichsweise geschwungen verläuft, sowie bei der das Ausatmungselement (9) mindestens bereichsweise gemeinsam mit dem Kupplungselement (12) den Ausströmkanal begrenzt, das Kupplungselement (12) über ein Gelenk (14) mit dem Maskengrundkörper (13) verbunden ist, das Ausatmungselement (9) innerhalb des Kupplungselementes (12) von mindestens einem Arretierungselement (30) fixiert ist, das mit einer Rastnase (31) einen zugeordneten Absatz (38) hintergreift und bei der der Absatz (38) im Bereich einer den Halterungsstutzen (17) mit dem Übergangssegment (16) verbindenden Überleitung (25) angeordnet ist,
**dadurch gekennzeichnet, daß** das Gelenk (14) kugelsegmentartig ausgebildet ist, daß das Ausatmungselement (9) von mehreren Arretierungselementen (30) fixiert ist und daß die Arretierungsflächen (39) der Rastnasen (31) relativ zu einer Längsachse (19) des Ausatmungselementes (9) derart geneigt angeordnet sind, daß eine Steigung ausgehend von einer Innenfläche (35) des Halterungsstutzens (17) in Richtung auf das Übergangssegment (16) bereitgestellt ist.

2. Beatmungsmaske nach Anspruch 1, **dadurch gekennzeichnet, daß** der Halterungsstutzen (17) relativ zum Maskengrundkörper (13) angewinkelt angeordnet ist.

3. Beatmungsmaske nach Anspruch 1 oder 2, **dadurch gekennzeichnet, daß** das Ausatmungselement (9) mindestens bereichsweise innerhalb des Halterungsstutzens (17) anordbar ist.

4. Beatmungsmaske nach einem der Ansprüche 1 bis 3, **dadurch gekennzeichnet, daß** das Ausatmungselement (9) außenseitig mindestens einen Außensteg (18) aufweist, der eine maximale Positioniertiefe des Ausatmungselementes (9) innerhalb des Kupplungselementes. (12) definiert.

5. Beatmungsmaske nach einem der Ansprüche 1 bis 4, **dadurch gekennzeichnet, daß** der spaltartige Ausströmkanal (36) zwischen einem Grundsegment (26) des Ausatmungselementes (9) und dem Halterungsstutzen (17) angeordnet ist.

6. Beatmungsmaske nach einem der Ansprüche 1 bis 5, **dadurch gekennzeichnet, daß** das Ausatmungselement (9) mit einem Verbindungsschlauch (5) koppelbar ist.

7. Beatmungsmaske nach einem der Ansprüche 1 bis 6, **dadurch gekennzeichnet, daß** das Grundsegment (26) des Ausatmungselementes (9) von mindestens zwei Verbindungsstegen (28) mit einem Halterungssegment (27) verbunden ist und daß sich zwischen den Verbindungsstegen (28) mindestens eine Ausströmöffnung (29) erstreckt.

8. Beatmungsmaske nach einem der Ansprüche 1 bis 7, **dadurch gekennzeichnet, daß** sich der Absatz (38) im wesentlichen konzentrisch relativ zur Längsachse (19) erstreckt.

9. Beatmungsmaske nach einem der Ansprüche 1 bis 8, **dadurch gekennzeichnet, daß** das Ausatmungselement (9) drehbeweglich innerhalb des Halterungsstutzens (17) geführt ist.

10. Beatmungsmaske nach einem der Ansprüche 1 bis 9, **dadurch gekennzeichnet, daß** das Ausatmungselement (9) relativ zum Halterungsstutzen (17) zentriert ist.

11. Beatmungsmaske nach einem der Ansprüche 1 bis 10, **dadurch gekennzeichnet, daß** die Außenstege (18) einen Anschlag für einen auf das Ausatmungselement (9) aufgeschobenen Verbindungsschlauch (5) bereitstellen.

## Claims

1. Respiratory mask for assisting the breathing of a patient, with a mask main body (13) connected via a coupling element (12) to an exhalation element (9) which bounds a gap-type outlet passage, and the coupling element (12) has a fitting connector (17) for positioning the exhalation element (9), and an articulated joint (14) connecting the coupling element (12) to the mask main body (13) is connected to the fitting connector (17) by means of a transition segment (16) and at least certain regions of the transition segment (16) extend in a curved arrangement, and the exhalation element (9) bounds the outlet passage jointly with the coupling element (12) in at least certain regions, and the coupling element (12) is connected to the mask main body (13) by means of an articulated joint (14), the exhalation element (9) is fixed inside the coupling element (12) by means of at least one locking element (30) with a catch lug (31) which engages behind a co-operating shoulder (38) and the shoulder (38) is disposed in the region of a transition fitting (25) connecting the fitting connector (17) to the transition segment (16),
**characterised in that** the articulated joint (14) is of the ball segment type, the exhalation element (9) is secured by several locking elements (30) and the locking surfaces (39) of the catch lugs (31) are inclined relative to a longitudinal axis (19) of the exhalation element (9), forming an inclination in the direction of the transition segment (16) starting from an internal face (35) of the fitting connector (17).

2. Respiratory mask as claimed in claim 1, **characterised in that** the fitting connector (17) is positioned at an angle relative to the mask main body (13).

3. Respiratory mask as claimed in claim 1 or 2, **characterised in that** at least certain regions of the exhalation element (9) can be disposed inside the fitting connector (17).

4. Respiratory mask as claimed in one of claims 1 to 3, **characterised in that** the exhalation element (9) has at least one external web (18) on the outside, which defines a maximum positioning depth of the exhalation element (9) inside the coupling element (12).

5. Respiratory mask as claimed in one of claims 1 to 4, **characterised in that** the gap-type outlet passage (36) is disposed between a base segment (26) of the exhalation element (9) and the fitting connector (17).

6. Respiratory mask as claimed in one of claims 1 to 5, **characterised in that** the exhalation element (9) can be coupled with a connecting hose (5).

7. Respiratory mask as claimed in one of claims 1 to 6, **characterised in that** the base segment (26) of the exhalation element (9) is connected to a retaining segment (27) by at least two connecting webs (28) and at least one outlet orifice (29) extends between the connecting webs (28).

8. Respiratory mask as claimed in one of claims 1 to 7, **characterised in that** the shoulder (38) extends essentially concentrically relative to the longitudinal axis (19).

9. Respiratory mask as claimed in one of claims 1 to 8, **characterised in that** the exhalation element (9) is guided in a rotating movement inside the fitting connector (17) .

10. Respiratory mask as claimed in one of claims 1 to 9, **characterised in that** the exhalation element (9) is centred relative to the fitting connector (17).

11. Respiratory mask as claimed in one of claims 1 to 10, **characterised in that** the external webs (18) constitute a stop for a connecting hose (5) pushed onto the exhalation element (9).

## Revendications

1. Masque respiratoire pour la ventilation d'un patient, dans lequel un corps de base de masque (13) dudit masque est relié, par l'intermédiaire d'un élément de couplage (12), à un élément d'expiration (9) qui limite un canal d'écoulement en forme de rainure, et dans lequel l'élément de couplage (12) présente une tubulure de fixation (17) pour le positionnement de l'élément d'expiration (9), ainsi que dans lequel une articulation (14), qui relie l'élément de couplage (12) au corps de base (13) dudit masque, est reliée à la tubulure de fixation (17) par un segment de transition (16), et dans lequel le segment de transition (16) s'étend en courbe, au moins sur une section, ainsi que dans lequel l'élément d'expiration (9) limite, en commun avec l'élément de couplage (12), le canal d'écoulement au moins sur une section, l'élément de couplage (12) est relié au corps de base de masque (13) par l'intermédiaire d'une articulation (14), l'élément d'expiration (9) est fixé à l'intérieur de l'élément de couplage (12) par au moins un élément d'arrêt (30) qui, avec un nez d'enclenchement (31), agrippe un talon (38) y associé, et dans lequel le talon (38) est disposé dans le domaine d'un passage (25) qui relie la tubulure de fixation (17) au segment de transition (16),
**caractérisé en ce que**
l'articulation (14) est conçue à la manière d'un organe à bille, **en ce que** l'élément d'expiration (9) est fixé par plusieurs éléments d'arrêt (30), et **en ce que** les surfaces d'arrêt (39) des nez d'enclenchement (31) sont disposés, inclinés par rapport à un axe longitudinal (19) de l'élément d'expiration (9), de sorte qu'une pente se trouve réalisée, à partir d'une surface intérieure (35) de la tubulure de fixation (17), en direction du segment de transition (16).

2. Masque respiratoire selon la revendication 1, **caractérisé en ce que** la tubulure de fixation (17) est disposée en formant un coude par rapport au corps de base de masque (13).

3. Masque respiratoire selon revendication 1 ou 2, **caractérisé en ce que** l'élément d'expiration (9) peut être disposé, au moins partiellement à l'intérieur de la tubulure de fixation (17).

4. Masque respiratoire selon l'une des revendications 1 à 3, **caractérisé en ce que** l'élément d'expiration (9) présente, sur le côté extérieur, au moins une patte extérieure (18) qui définit une profondeur de positionnement maximale de l'élément d'expiration (9) à l'intérieur de l'élément de couplage (12).

5. Masque respiratoire selon l'une des revendications 1 à 4, **caractérisé en ce que** le canal d'écoulement en forme de rainure (36) est disposé entre un segment de base (26) de l'élément d'expiration (9) et la tubulure de fixation (17).

6. Masque respiratoire selon l'une des revendications 1 à 5, **caractérisé en ce que** l'élément d'expiration (9) peut être couplé avec un tuyau de liaison (5).

7. Masque respiratoire selon l'une des revendications 1 à 6, **caractérisé en ce que** le segment de base (26) de l'élément d'expiration (9) est relié, par au moins deux pattes de liaison (28), à un segment de fixation (27), et que, entre les pattes de liaison (28), s'étend au moins une ouverture d'écoulement (29).

8. Masque respiratoire selon l'une des revendications 1 à 7, **caractérisé en ce que** le talon (38) s'étend de manière sensiblement concentrique par rapport à l'axe longitudinal (19).

9. Masque respiratoire selon l'une des revendications 1 à 8, **caractérisé en ce que** l'élément d'expiration (9) est guidé, mobile en rotation, à l'intérieur de la tubulure de fixation (17).

10. Masque respiratoire selon l'une des revendications 1 à 9, **caractérisé en ce que** l'élément d'expiration (9) est centré par rapport à la tubulure de fixation (17).

11. Masque respiratoire selon l'une des revendications 1 à 10, **caractérisé en ce que** les pattes extérieures (18) forment une butée pour un tuyau de liaison glissé sur l'élément d'expiration (9).
